(19) Europäisches Patentamt
European Patent Office
Office européen des brevets

(11) Publication number: **0 000 102**
**B1**

(12) # EUROPEAN PATENT SPECIFICATION

(45) Date of publication of patent specification: **09.09.81**

(21) Application number: **78300034.2**

(22) Date of filing: **12.06.78**

(51) Int. Cl.³: **G 01 N 33/54,** C 07 G 7/00, A 61 K 39/385

(54) **Immunologic compositions, methods for preparing them and methods for conducting haemagglutination tests.**

(30) Priority: **14.06.77 US 806563**

(43) Date of publication of application:
**20.12.78 Bulletin 78/1**

(45) Publication of the grant of the European patent:
**09.09.81 Bulletin 81/36**

(84) Designated Contracting States:
**BE CH DE FR NL SE**

(56) References cited:
**DE - A - 1 907 077**
**FR - A - 2 187 909**
**US - A - 3 914 400**
**US - A - 3 991 174**

(73) Proprietor: **AMERICAN HOME PRODUCTS CORPORATION**
**685, Third Avenue**
**New York, New York 10017 (US)**

(72) Inventor: **Hirsch, Michael Allen**
**747, Twin Rivers Drive**
**Northeast Windor Township New Jersey (US)**
Inventor: **Irvine, Douglas Stalker**
**320, Coolbrook Road**
**Rosemere, Quebec (CA)**
Inventor: **Krupey, John**
**2645, Raudot Street**
**Montreal, Quebec (CA)**

(74) Representative: **Brown, Keith John Symons et al,**
**c/o John Wyeth & Brother Limited Huntercombe Lane South**
**Taplow Maidenhead, Berkshire SL6.OPH. (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 000 102 B1

# Immunologic compositions, methods for preparing them and methods for conducting haemagglutination tests

This invention relates to immunologic compositions and antiserum compositions which may be used with the immunologic compositions.

The development of reliable immunologic compositions useful as reagents in immunoassays to determine the presence or absence of antigens or antibodies in a test fluid has and continues to be a sought after goal. One approach which has received a great deal of attention has been the development of reagents for haemagglutination and haemagglutination inhibition systems. These systems employ red blood cells also known as erythrocytes which, for use, are joined with antigenic or antibody substances so as to provide an indicator system which can be used to detect the antibody or the antigenic substance itself.

An early development in haemagglutination inhibition or passive haemagglutination tests was described by Wide and Gemzell, Acta Endocr., *35* 261 (1960). They established that the red blood cells (Rbc) coated by or attached to the antigen must be stabilised to be useful in haemagglutination inhibition tests, otherwise the red blood cells would need to be fresh daily. While Wide *et al* stabilised the erythrocytes before attachment of the antigen, others have stabilised following attachment of the antigen.

Stabilisation of Rbc was originally described by Boyden using formaldehyde, J. Exp. Med., *93* 107 (1951) and the concept was extended by Wide, Acta Endocr., supp. 70, 41 (1962), when following pretreatment of formaldehyde treated Rbc with tannic acid, it was shown that hCG could be adsorbed onto the cell surface of the Rbc.

Ling, Brit. J. Haemat., *7* 229 (1961), also showed that pyruvic aldehyde could beneficially replace formaldehyde and that pretreatment with tannic acid was not necessary for antigen attachment. However, for stabilisation, Ling decreed that 48 hours at +4°C was necessary. But, the coated erythrocytes are unstable and are also time consuming to prepare.

One approach to improving stability and also of obtaining erythrocytes with higher haemagglutination titres was disclosed in U.S. Patents 3,714,345; 3,715,427 and 3,925,541. There the erythrocytes were treated sequentially with pyruvic aldehyde and then formaldehyde for periods in excess of 12 hours for each treatment prior to coating the thus stabilised erthrocytes with an antigen or antibody. The double aldehyde treated erythrocytes were in some cases further subjected to a lengthy freeze-thaw cycle at very low temperatures.

Another approach to obtain antigen sensitised erythrocytes was through the use of bivalent reagents as coupling agents between antigens and erythrocytes, e.g. bis diazotized benzidines illustrated in U.S. Patent 3,236,732; various diols and quinones described in U.S. Patent 3,322,634; toluene-2,4-diisocyanates described in Immunochemistry, *1,* 43 (1964). Since even the coupling coated erythrocytes are still readily susceptible to decomposition, formaldehyde treatment was usually employed prior to coating with antigen or simultaneously as described in U.S. Patent 3,987,159 where the coupling agent employed was glutaraldehyde. In still a different approach described in U.S. Patent 3,991,175, glutaraldehyde as also utilized as a coupling agent but gelatin was used to stabilize the composition.

The various compositions and methods already noted and numerous others suffer individually from various disadvantages such as instability, lack of reproducibility, false positive results, low haemagglutination titres, cost or time of preparation, and others.

It has now been found that the compositions and methods of this invention, demonstrated in part by use in haemagglutination tests, provide a superior grade of stabilised sensitized cells. The compositions of this invention have higher haemagglutination titres, are stable for long periods of time, and give reproducible patterns and results. Another advantage of the herein disclosed invention is the stability of results obtained. For example, in utilizing a commercially available reagent the haemagglutination patterns change with time. Thus, in a pregnancy test, a negative result, i.e. complete haemagglutination within 2 hours, will change over 24 hours to show a quasi-inhibition pattern; results recorded from 2—24 hours at room temperature will therefore indicate a significant number of false positive results. However, utilizing the composition and methods of this invention, results are obtained within 2 hours of testing and will not change for about 11 days thereafter or more. Other advantages will become apparent as the description of the invention unfolds.

Accordingly the present invention provides an immunologic composition of pyruvic aldehyde or dimethylsuberimidate stabilised erythrocytes sensitised with a polypeptide or glycoprotein antigen selected from chorionic gonadotrophin pregnant mare's serum gonadotrophin, carcino embryonic antigen, luteinising hormone, follicle stimulating hormone, human menopausal gonadotrophin and thyroid stimulating hormone, said stabilised erythrocytes being coupled to said antigen with a bifunctional molecule selected from glutaraldehyde, glyoxal, succinaldehyde, hexamethylene diisocyanate, toluene 2,4-diisocyanate, a bifunctional imido ester, (e.g. diethyl malonimidate dihydrochloride or dimethyl suberimidate), cyanuric chloride, tetrazotized o-anisidine, and water soluble carbodiimide (e.g. 1-ethyl-3-(3-dimethylaminopropyl)carbodiimide) with the provisos that when the erythrocytes are dimethylsuberimidate stabilised the bifunctional molecule is other than dimethyl suberimidate, and that when the erythrocytes are pyruvic aldehyde stablised the bifunctional molecule is glutaraldehyde, glyoxal, succinaldehyde, hexamethylene diisocyanate, toluene 2,4- diisocyanate or a bifunctional imido ester.

Preferably the erythrocytes are pyruvic aldehyde stabilised. Thus in a preferred aspect the present invention provides an immunologic composition comprising pyruvic aldehyde stabilised erythrocytes sensitized with a polypeptide or glycoprotein antigen selected from chorionic gonadotrophin, pregnant mare's serum gonadotrophin, carcino embryonic antigen, luteinizing hormone, follicle stimulating hormone, human menopausal gonadotrophin and thyroid stimulating hormone, said stabilised erythrocytes being coupled to said antigen with a bifunctional molecule selected from glutaraldehyde, glyoxal, succinaldehyde, hexamethylene diisocyanate, toluene-2,4-diisocyanate, bifunctional imido esters including diethylmalonimidate hydrochloride and dimethyl suberimidate. It is preferred to use as the bifunctional molecule for coupling glutaraldehyde, hexamethylene diisocyanate or dimethyl suberimidate.

When the erythrocytes are dimethyl suberimidate stabilised the coupling agent or bifunctional molecule can be glutaraldehyde, glyoxal succinaldehyde, hexamethylene diisocyanate, toluene 2,4-diisocyanate, a bifunctional imido ester other than dimethyl suberimidate, cyanuric chloride, tetrazotized o-anisidine or a water soluble carbodiimide such as 1-ethyl-3-(3-dimethylaminopropyl)carbodiimide.

In a further aspect the invention provides a method for preparing immunologic compositions of animal erythrocytes sensitized with antigens useful in passive haemagglutination tests which comprises:

a) collecting animal blood directly into an isotonic sterile anticoagulant so that a final ratio of blood to anticoagulant is from 0.8:1 to 1.2:1 of the resulting mixture wherein the erythrocytes concentration packed cell volume of said blood is from 15% to 25%.

b) separating the erythrocytes from said mixture by centrifugation;

c) washing said erythrocytes with normal saline and then suspending said erythrocytes in normal saline at a concentration of 10 to 30%:

d) mixing the suspension of erythrocytes with a mixture of pyruvic aldehyde, neutral buffered solution and normal saline to obtain stabilization mixture;

e) incubating the stabilization mixture of step (d) at 30 to 35°C for one to five hours, and collecting the stabilised erythrocytes by centrifugation followed by washing with normal saline;

f) suspending the stabilised erythrocytes in a neutral buffered solution of an antigen selected from chorionic gonadotrophin, pregnant mare's serum gonadotrophin, carcino embryonic antigen, luteinizing hormone, follicle stimulating hormone, human menopausal gonadotrophin and thyroid stimulating hormone;

g) forming a sensitization mixture by thorough agitation of the suspension of stabilized erythrocytes-antigen with a solution of a bifunctional molecule in saline, the bifunctional molecule being selected from glutaraldehyde, glyoxal, succinaldehyde, hexamethylene diisocyanate, toluene 2,4-diisocyanate and a bifunctional imido ester;

h) collecting the sensitized erythrocytes by centrifugation and subsequently washing them with neutral buffered saline;

i) suspending the sensitized erythrocytes in a neutral buffered saline containing normal animal serum wherein the complement of said serum has previously been fixed; said erythrocytes being mammalian, avian or reptile erythrocytes.

The above mentioned method may be employed to prepare the immunologic compositions of the invention containing pyruvic aldehyde stabilised erythrocytes. Immunologic compositions containing dimethyl suberimidate stabilised erythrocytes may be prepared in an analogous manner.

A preferred manner of carrying out the method of the invention in which a composition of stabilised red blood cells (erythrocytes) sensitized with a polypeptide or glycoprotein such as, human chorionic gonadotrophin (hCG) is prepared as one component in a two component reagent system is described below. Stabilisation of the erythrocytes may be first accomplished by taking freshly drawn whole blood, generally from sheep, although turkeys, goats or horses can be used, and immediately mixing it with an isotonic sterile anticoagulant, such as Alsever's solution, so that a final ratio of blood to anticoagulant is from 0.8:1 to 1.2:1 preferably 1;1, of the resulting mixture. The erythrocytes are then separated by centrifugation and washed with physiological saline. Pyruvic aldehyde reagent is prepared in physiological saline. A neutral buffered solution, preferably phosphate buffer, and a suspension of washed erythrocytes in saline are added to the pyruvic aldehyde reagent. The suspension is then incubated at 30 to 55°C for one to five hours, preferably at 37°C for three hours, with occasional agitation. Subsequently the stabilised red blood cells are thoroughly washed with saline. The pyruvic aldehyde stabilised cells (PAC) may then be stored at 4°C as a 10% suspension in saline containing 0.1% sodium azide as a preservative, for at least a year, if desired. To sensitize the stabilised cells with hCG, the pyruvic aldehyde treated cells are washed with dilute saline and then suspended in a neutral buffered solution, preferably phosphate buffer, containing hCG. To this suspension is added glutaraldehyde in normal saline. The mixture is then incubated, for example, at room temperature for about 2 hours under constant agitation and the cells are removed by centrifugation and washed not less than 3 times with neutral buffered saline, preferably phosphate buffered saline. Optionally, the sensitized cells are suspended in a neutral buffered saline, preferably phosphate buffered saline, containing as little as 0.2% normal rabbit serum in which complement had previously been fixed by heating at 56°C for about 30 minutes and from which non-specific agglutinins, interfering proteins, had previously been removed by serial adsorption with an equal volume of pyruvic aldehyde stabilised cells. Alternatively, normal serum from goats, horses or sheep may be used. Sensitized cells may be stored at 4°C. Again alternatively, in place of normal rabbit serum, one could use any properly treated stabilisation agent including properly adsorbed, complement fixed serum proteins. Illustrative of such stabilisation agents are gelatin, dextrans, polyvinyl pyrrolidone, albumins and water soluble carboxymethyl celluloses.

The hCG used for sensitization of the stabilised cells, illustrated above may be of various grades of purity. For example, material which assays, biologically, at approximately 2,500 I.U./mg may be employed. Alternatively, more purified hCG preparations, for example, a preparation assaying at approximately 10,000 I.U./mg or 14,000—16,000 I.U./mg may be utilized in the sensitization of the aforementioned stabilised red blood cells. Again the beta sub-unit of hCG may also be utilized as the antigen on the red blood cell. It is generally preferable to use the more purified hCG in the stabilised, sensitized erythrocyte compositions of this invention.

While the invention has been illustrated primarily using mammalian erythrocytes, such as those from sheep. these stabilised, sensitized erythrocytes may also be prepared from avian or reptilian red blood cells, which being nucleated settle more rapidly than mammalian erythrocytes. In this way the results from haemagglutination tests may be obtained within 30 minutes or sooner. The speed of the haemagglutination reaction is proportional to the buoyant density of the nucleated cell; thus as the buoyant density increases, less time is required to obtain results. Furthermore, whereas the invention was illustrated using Alsever's solution as the isotonic sterile anticoagulant, the freshly drawn whole blood may be optionally mixed with heparin sodium EDTA, or sodium oxalate.

The described stabilised, sensitized erythrocyte compositions together with their antiserum compositions are most useful in haemagglutination and haemagglutination inhibition (passive haemagglutination) methods for determining the presence or absence of a particular antigen or antibody in the test fluid being examined, usually a body fluid such as urine or serum. Thus determining the presence or absence of a pregnant condition in a female animal can be based on a haemagglutination method for determining the presence of chorionic gonadotrophin which comprises mixing the erythrocyte composition of this invention with a chorionic gonadotrophin antiserum and with the urine of test subject and, following a suitable incubation period, visually observing the results, whereby if said gonadotrophin is not present agglutination of the erythrocytes occurs upon standing whereas if said gonadotrophin is present, no such agglutination occurs. A method for determining the presence of luteinizing hormone in a woman and thereby the day of ovulation may also be done utilizing the compositions of this invention wherein a urine sample is concentrated by ultrafiltration and luteinizing hormone detected by immunological means, the antigen being human chorionic gonadotrophin, as described in U.S. Patent Specification No. 4,123,510. Similarly, pregnancy in a mare may be determined utilizing as the antigen pregnant mare's serum gonadotrophin and a composition utilizing carcino embryonic antigen would be useful in a blood test for cancer. Other uses for the composition of this invention are described therein.

One use of the composition of the invention comprises a method for determining the presence or absence of human chorionic gonadotrophin and a human urine sample which comprises mixing the composition of the invention in which the antigen is human chorionic gonadotrophin and the erythrocytes are mammalian, avian or reptile pyruvic aldehyde stabilised erythrocytes with a highly purified hCG antiserum and with ultrafiltration concentrated human urine whereby if hCG is not present agglutination of the erythrocytes occurs upon standing whereas if hCG is present no such agglutination occurs.

As illustrated below utilizing as the antigen, hCG, it is hypothesized that the superior properties of the compositions of this invention are due to the coupling of the stabilised erythrocytes to the hCG by means of a chemical reaction between the amino functions of the erythrocytes and the hCG and the difunctional glutaraldehyde. The suggested course of reaction of glutaraldehyde is as follows:

(a) Aldol condensations:

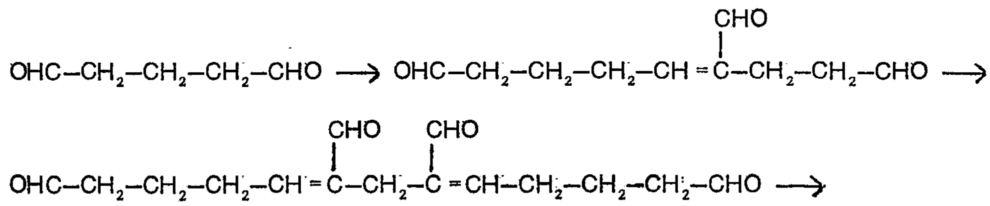

"X"

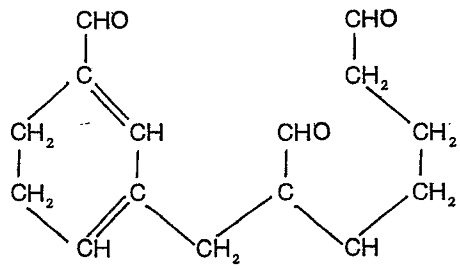

+ etc.

(b) Cross-linking reactions:

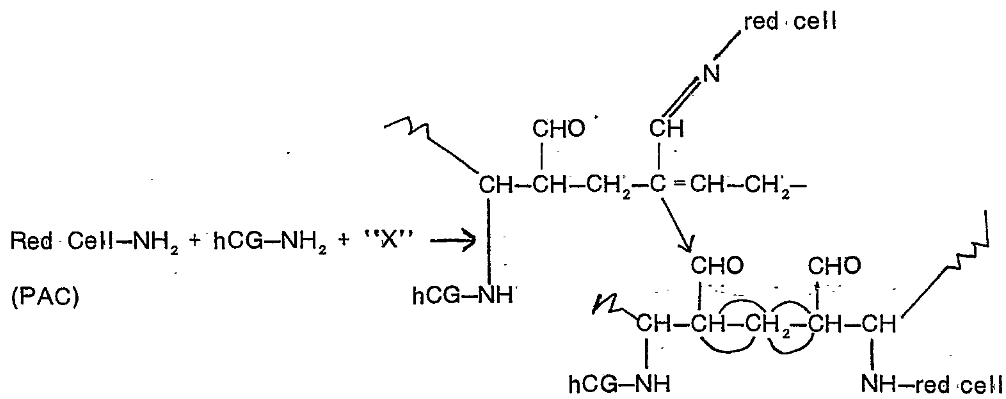

The reaction with hexamethylene diisocyanate is thought to proceed in the following fashion:

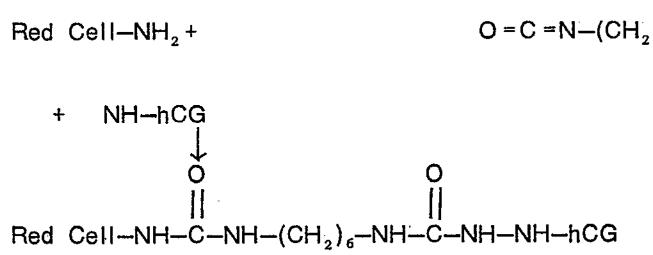

urea derivative

In order to conduct the passive haemagglutination tests contemplated by this invention it is necessary to prepare an antiserum composition to be used in conjunction with the antigen sensitized erythrocytes previously described. Depending on the test hCG which assays biologically as low as 2500 I.U./mg may be employed. Alternatively, more purified hCG preparations that assay at approximately 10,000 I.U./mg, 15,000 I.U./mg or higher may

be employed. It is preferred to utilize highly purified hCG and most preferred to use the $\beta$-sub-unit of hCG in preparing the antiserum of this invention to obtain greater specificity. An example of highly purified $\beta$-hCG and a method of producing same is reported in our copending European application No. 0000103 filed concurrently herewith.

The antiserum compositions are generally prepared by immunizing a host animal with the desired antigen thereby producing antibodies to that antigen which may be obtained in the serum separated from the host animal. A difficulty in the past has been that the hCG antigen has had other antigen impurities present, thus undesirable antibodies were also produced. A haemagglutination method utilizing such an antiserum composition would thus possess undesirable cross reactivity.

The antisera composition of this invention is dilute diether in microtitre slides or in a tube system titration to determine the maximum dilution of antisera which can still agglutinate the red blood cell. The dilution is then adjusted when utilized in a method for determining pregnancy so that the anti-hCG serum has a sensitivity to hCG of about 100—150 m I.U./test. The acceptable antisera will also have a cross reactivity of less than 25% particularly against other glycoprotein hormone antigens. Thus an antisera sensitive to 100 m I.U./test of hCG would at the same time have a sensitivity to 400 m I.U./test or more of hLH or hMG. This highly specific antiserum composition obtained using highly purified hCG as the antigen, will detect early pregnancy via the presence of small amounts of hCG and also minimises the possibility of obtaining false positives in the passive haemagglutination inhibition test. A method and apparatus for use of the herein described reagents is described, U.S. Patent Specification No. 4,123,509, U.K. Patent Specifications Nos. 1537538 and 1537537 and in U.S. Patent No. 4,033,723.

While the compositions of this invention may be utilized in aqueous compositions, it is preferred to lyophilize these compositions for use in the particular haemagglutination method under consideration. The compositions may be lyophilized into two separate pellets by methods known to those skilled in the art such as that described in U.S. Patent 3,862,302. That disclosure, however, does not teach how to make lyophilized compositions containing in either the antiserum or the stabilised erythrocytes enough buffer for complexing the interfering calcium ions present in urine. Rather it involves the use of a third lyophilized pellet containing the necessary buffers or as in a commercially available pregnancy test a separate buffer compositions which is reconstituted at the time of its use.

The compositions of this invention may also be lyophilized into a single layered cake, as described in U.S. Patent 3,269,905. Also while a suitable chelating agent may be included within the lyophilized compositions of this invention to remove quantities of calcium present in a urine test sample, the compositions of this invention without added chelating agent may be utilized with urine and/or ultra-concentrated urine which has been passed through a filter in which is placed a styrene divinyl benzene copolymer containing imide acetate functional groups (CHELEX 100 or DOWEX CHELATING RESIN A—1).

The physical characteristics of the vial in which the immunologic reagents e.g., the Rbc preparation described in this invention together with a suitable antiserum and appropriate buffers which are to be used in passive haemagglutination tests, must be properly defined. Pyrex culture tubes have been subjected to various physical pretreatments prior to utilization of the immunologic reagents, described herein in passive haemagglutination tests. We have found that round bottomed siliconized glass tubes having an inner diameter of 12—16 mm are preferable in order to obtain reproducible flocculation times and reproducible agglutination patterns.

The invention is further illustrated in the following examples:

## Example 1

Whole sheep blood was collected directly into Alserver's solution so that the final ratio of sheep blood to Alserver's solution was 1:1 (For the purpose of this invention it has been established that the Rbc concentration or packed cell vlume (pcv) of such a mixture should be in the range of 15—25% (i.e. a hematocrit of 30—50%) and preferably, 15 to 25% or more preferably 22%. The cells from 100 ml of such a mixture were collected by centrifugation and washed three times with normal, saline solution, i.e. 0.9% (w/v) sodium chloride in distilled water. The preferred volume of saline per wash is 320 ml but may be in the range of 100—500 ml. After washing, the cells are suspended in normal saline solution at a concentration of 20%. A mixture of pyruvic aldehyde (64 ml of 25% aqueous solution) and normal saline (120 ml) was adjusted to pH 7.0 using a 10% w/v aqueous sodium carbonate solution. To this mixture of pyruvic aldehyde and saline was added the above-mentioned cell suspension (100 ml at 20%), followed by phosphate buffer (28 ml., 0.15 M, pH 8.0). This stabilization mixture was then incubated at 37°C for 3 hours, the mixture being shaken vigorously once every 30 minutes.

The cells were then collected by centrifugation and washed 4 times with normal saline solution utilizing preferably 320 ml of saline per wash. The stabilised cells may then be stored as a suspension, preferably 10%, in normal saline containing, for example, 0.1% sodium azide as a preservative. This is stable for at least a year at +4°C.

Example 2

Stabilized cells as described in Example 1 (10 ml of about a 10% suspension) were collected by centrifugation and washed 3 times with normal saline solution, preferably 40 ml for each wash. Alternatively, one may of course use unstored cells directly from Example 1. These washed cells were then suspended in a solution of hCG (100 $\mu$g in 100 $\mu$l of 0.5 M phosphate buffer) contained in 0.15 M phosphate buffer (8.65 ml, pH 7.4). To this suspension was added a glutaraldehyde reagent (1.75 ml), prepared by diluting a 25% aqueous solution of glutaraldehyde (1 ml) in normal saline (9 ml).

The sensitization mixture was thoroughly agitated and then gently mixed for 2 hours at room temperature. The cells were then collected by centrifugation and washed, preferably 4—5 times, with 0.15 M phosphate buffered saline (pH 7.4).

The sensitized cells are then suspended in 0.15 M phosphate buffered saline (39 ml, pH 7.4) containing 0.2% of normal rabbit serum in which complement had previously been fixed by incubating at 56°C for 30 minutes and from which non-specific agglutinins had been adsorbed by triple treatment with stabilised cells (Example 1). The removal of non-specific agglutinins is illustrated in Example 3.

Example 3

Prior to use for the removal of non-specific agglutinins from normal rabbit serum, stabilised cells as prepared in Example 1, were washed with normal saline (5 volumes saline: 1 volume of 10% cell suspension) and resuspended in saline solution at 10%. Equal volumes of rabbit serum and a 10% suspension of stabilised red blood cells were then incubated at room temperature for 30 minutes. The cells were collected by centrifugation, discarded and the process repeated 2 more times, using collected cells as opposed to a 10% suspension.

Alternatively, the last adsorption may be allowed to proceed at +4°C for 18 hours. Under the conditions described in this example, the normal rabbit serum has been diluted 1:1 with saline. It should be understood, however, that for this adsorption it is possible to utilize collected cells as opposed to 10% suspension which would not result in a dilution of the normal rabbit serum.

Example 4

Utilizing the methods of Example 2, but with pregnant mare's serum gonadotrophin as the antigen, a stable sensitized composition was also prepared and satisfactorily evaluated.

Example 5

Lyophilized cakes were prepared as follows. A suspension of the stabilised, sensitized red blood cells (S—PAGC) was washed about 3 times with a 0.15 M phosphate buffer solution adjusted to a pH of 7.4. The washed S—PAGC were then resuspended as a 0.415% v/v suspension in lyophilization buffer, LB, (LB used was 10 g sucrose, 10 ml of 1% merthiolate, 10 ml of NRS triply adsorbed, and q.s. to 1 litre with 0.15 M phosphate buffered saline containing about 0.2% EDTA to a pH of 7.0). 300 $\mu$l of the cell suspension were then pipetted into diferent siliconized vials already in a test tube rack, and the rack containing the vials was immersed in an acetone-dry ice bath at about −70°C and frozen. Dilutions of antisera and NRS, (which had been pretitrated and adjusted to give a predetermined sensitivity) were made with LB such that the proper concentration of antisera in NRS is contained in a 200 $\mu$l aliquot, was then pipetted onto the frozen cell layers in the bath.

The frozen reagents may then be stored at about −100°C until lyophilization or lyophilized immediately. Lyophilization of these reagents was then done in a freeze drier for at least 18 hours at about 75—200 microns Hg, while gradually reached ambient temperatures. The vacuum was not permitted to be less than 75 microns to avoid spontaneous haemagglutination. The lyophilized reagents when stored at 4°C are stable for at least 9 months.

Example 6

Lyophilized pellets were prepared as follows: A suspension of S—PAGC was washed as in Example 5 and then resuspended as a 2.5% suspension in LB concentrated by a factor of 5 in all its components. This latter suspension was constantly agitated while a proportioning pump repeatedly delivered about 50 $\mu$l of the S—PAGC suspension into a liquid $N_2$ bath at −196°C and the pellets were harvested in a petri dish.

The antisera pellets were prepared similarly but may optionally have incorporated within the LB 2.5% of polyvinyl pyrrolidone or dextran. The pellets may be stored as the cakes are stored in Example 4 or transferred immediately to the freeze drier. Pellets have been dried satisfactorily at about 50—200 microns Hg for about 18 hours, with the temperature finally reaching room temperature or in some cases 35°C. The pellets were stored in a dessicator at room temperature until packing into siliconized vials. These pellets have been found to maintain their sensitivity and physical characteristics in long term stability studies at both 30°C and 4°C.

Example 7

Pretreatment of tubes with silicone is most beneficial as exemplified in Example 8 below. The method used for pretreatment with silicone is described under e), whilst other pretreatments, used for comparative purposes, are described in a) to d).

a) Untreated tubes used directly from the manufacturer.

b) Pyrolyzed tubes prepared by heating at 560°C (not more than 5 minutes).
c) BSA coated tubes
   i) washed in 1% (w/v) bovine serum albumin with agitation, 20 sec.
   ii) rinse in running distilled water, 3 times
   iii) dry overnight at 60°C.
d) BSA-coated acid washed tubes
   i) chromic-sulfuric acid wash, overnight soak.
   ii) rinse in running tap water, 3 times.
   iii) soak in 95% ethanol — one hour, 2 changes.
   iv) rinse in running tap water, 2 times.
   v) rinse in running distilled water, 3 times.
   vi) dry overnight at 100°C.
   vii) wash in 1% BSA with agitation, 20 sec.
   viii) rinse in running distilled water, 3 times.
   ix) dry overnight at 60°C.
e) Siliconized tubes
   i) wash in 1% (v/v) SILICLAD (Clay Adams), a silane in aqueous alcohol solution, with agitation, 20 sec.
   ii) rinse in running distilled water, 6 times.
   iii) dry overnight at 100°C.

Example 8

The following results were observed with the pretreatment tubes of Example 7.

i) Untreated, pyrolized, BSA-treated and acid washed BSA-treated vials lead to collapsed cell matts, or matts of rough appearance, when a haemagglutination pattern should result using the reagents of this invention.

ii) With the vials pretreated as in (i) above, the transition from haemagglutination to inhibition of haemagglutination, is irregular.

iii) Vials pretreated with silicone demonstrate smooth haemagglutination patterns.

iv) In vials pretreated with silicone, the transition from haemagglutination to inhibition of haemagglutination in the presence of increasing amount of antigen, is regular and the degree to which haemagglutination is inhibited is proportional to the increasing amount of antigen present.

A method for preparing the antiserum composition of this invention is illustrated in the following Example 9.

Example 9

*Antigen* β-hCG subunit

*Initial Challenge Solution (ICS)*

| | |
|---|---|
| Tubercle bacillus | 5.0 mg |
| β-hCG | 200 mg |
| saline | 2 ml |
| complete Freund s adjuvant | 2 ml |

*Subsequent Challenge Solution (SCS)*

| | |
|---|---|
| β-hCG | 1.0 mg |
| saline | 5 ml |
| complete Freund's adjuvant | 5 ml |

*Booster Solution (BS)*

| | |
|---|---|
| β-hCG | 100 μg |
| saline | 1 ml |

Each of the ICS and SCS are homogenized well until the suspensions are thick and creamy.

*Immunization Protocol*

Day 1 — Three month old virgin female New Zealand white rabbits are injected with 2.0 ml of "ICS" at 30—50 intradermal sites. Five hundred μl of crude *Bordetella pertussis* vaccine is injected at a separate intradermal site.
Day 14 — Each rabbit is bled from the marginal ear vein.
Day 15 — Each rabbit is injected with a total of 1.0 ml "SCS" in the hind foot pads.
Days 22, 29, 36, 43 — Each rabbit is injected with a total of 1.0 ml "SCS" in multiple intradermal sites.
Day 49 — Each rabbit is injected with 1.0 ml "BS" intravenously in the marginal ear vein.
Day 56 — Each rabbit is bled from the marginal ear vein.

Thereafter, one ml of "BS" is injected intravenously every 6 weeks and the rabbits bled 5—7 days after each booster. The total time necessary to produce an antiserum using this combined multiple intradermal site-foot pad method of immunization will vary and is considered complete when the antibody titre which is constantly being monitored reaches a plateau.

The antiserum is separated from the rabbit blood and complement may be fixed and the suspension adsorbed as described with the S—PAGC composition in Example 2 and Example 3, a single adsorption usually being sufficient. Undiluted antisera or diluted antisera may be stored preferably at −100°C in small aliquots.

## Claims

1. An immunologic composition of pyruvic aldehyde or dimethylsuberimidate stabilised erythrocytes sensitized with a polypeptide or glycoprotein antigen selected from chorionic gonadotrophin, pregnant mare's serum gonadotrophin, carcino embryonic antigen, luteinizing hormone, follicle stimulating hormone, human menopausal gonadotrophin and thyroid

stimulating hormone, said stabilized erythrocytes being coupled to said antigen with a bifunctional molecule selected from glutaraldehyde, glyoxal, succinaldehyde, hexamethylene diisocyanate, toluene 2,4-diisocyanate, a bifunctional imido ester, cyanuric chloride, tetrazotized o-anisidine, and a water soluble carbodiimide, with the provisos that when the erythrocytes are dimethyl suberimidate stabilised the bifunctional molecule is other than dimethylsuberimidate and that when the erythrocytes are pyruvic aldehyde stabilised the bifunctional molecule is glutaraldehyde, glyoxal, succinaldehyde, hexamethylene diisocyanate, toluene 2,4-diisocyanate or a bifunctional imido ester.

2. A composition as claimed in Claim 1 wherein the polypeptide or glycoprotein antigen is human chorionic gonadotrophin.

3. A composition as claimed in Claim 1 or 2 wherein the erythrocytes are pyruvic aldehyde stabilised.

4. A composition as claimed in any one of Claims 1 to 3 wherein the bifunctional molecule is glutaraldehyde, hexamethylene diisocyanate or dimethyl suberimidate.

5. A composition as claimed in any one of Claims 1 to 4 in which the composition is lyophilized.

6. A composition as claimed in any one of Claims 1 to 5 wherein the antigen sensitized erythrocytes have been treated with phosphate buffered saline containing a serum protein in which complement had previously been fixed and from which non-specific agglutinins had previously been removed by serial adsorption with pyruvic aldehyde stabilized erythrocytes.

7. A composition as claimed in any one of Claims 1 to 5 wherein the antigen sensitized erythrocytes have been treated with phosphate buffered saline containing a member selected from gelatin, dextrans, polyvinyl-pyrrolidine, and water soluble carboxymethyl celluloses which had previously been treated by adsorption with pyruvic aldehyde stabilized erythrocytes.

8. A method for preparing immunologic compositions of animal erythrocytes sensitized with antigens useful in passive haemagglutination test which comprises:

a) collecting animal blood directly into an isotonic sterile anticoagulant so that a final ratio of blood to anticoagulant is from 0.8:1 to 1.2:1 of the resulting mixture wherein the erythrocyte concentration packed cell volume of said blood is from 15% to 25%;

b) separating the erythrocytes from said mixture by centrifugation;

c) washing said erythrocytes with normal saline and then suspending said erythrocytes in normal saline at a concentration of 10 to 30%;

d) mixing the suspension of erythrocytes with a mixture of pyruvic aldehyde, neutral buffered solution and normal saline to obtain stabilization mixture;

e) incubating the stabilization mixture of step (d) at 30 to 55°C for one to five hours, and collecting the stabilized erythrocytes by centrifugation followed by washing with normal saline;

f) suspending the stabilized erythrocytes in a neutral buffered solution of antigen selected from chorionic gonadotrophin, pregnant mare's serum gonadotrophin, carcino embryonic antigen, luteinizing hormone, follicle stimulating hormone, human menopausal gonadotrophin and thyroid stimulating hormone;

g) forming a sensitization mixture by thorough agitation of the suspension of stabilized erythrocytes antigen with a solution of a bifunctional molecule in saline, the bifunctional molecule being selected from glutaraldehyde, glyoxal, succinaldehyde hexamethylene diisocyanate, toluene 2,4-diisocyanate and a bifunctional imido ester;

h) collecting the sensitized erythrocytes by centrifugation and subsequently washing them with neutral buffered saline;

i) suspending the sensitized erythrocytes in neutral buffered saline containing normal animal serum wherein the complement of said serum has previously been fixed, said erythrocytes being mammalian, avian or reptile erythrocytes.

9. A method as claimed in Claim 8 for preparing a composition as claimed in Claim 3 wherein the antiger is human chorionic gonadotrophin.

10. A method as claimed in Claim 8 or 9 wherein the composition is lyophilized.

11. A method for determining the presence or absence of human chorionic gonadotrophin in a human urine sample characterised in that the composition of Claim 3 in which the antigen is human chorionic gonadotrophin and the erythrocytes are mammalian, avian or reptilian erythrocytes, is mixed with a highly purified hCG antiserum and with ultrafiltration concentrated human urine whereby if hCG is not present agglutination of the erythrocytes occurs upon standing whereas if hCG is present no such agglutination occurs.

12. An antiserum composition to hCG for use in a method as claimed in Claim 11 which has been prepared using a highly purified hCG as the immunizing antigen, said composition having a cross reactivity to other glycoprotein hormone antigens of less than 25 percent.

**Revendications**

1. Une composition immunologique d'érythrocytes stabilisés à l'aldéhyde pyruvique ou au subérimidate de diméthyle, sensibilisés avec un antigène polypeptidique ou glycoprotéinique choisi entre la gonadotrophine chorionique, la gonadotrophine de sérum de jument gravide, l'antigène du carcinome embryonnaire, l'hormone lutéinisante, la folliculostimuline, la gonadotrophine humaine de la ménopause et l'hormone stimulant la thyroïde,

ces érythrocytes stabilisés étant couplés audit antigène avec un molécule bifonctionnelle choisie entre la glutaraldéhyde, le glyoxal, le succinaldéhyde, l'hexaméthylène-diisocyanate, le 2,4-diisocyanto-toluène, un imido-ester bifonctionnel, le chlorure cyanurique, la o-anisidine tétrazotée et un carbodiimide hydrosoluble, à condition que lorsque les érythrocytes sont stabilisés au subérimidate de diméthyle, la molécule bifonctionnelle soit autre que le subérimidate de diméthyle et que lorsque les érythrocytes sont stabilisés à l'aldéhyde pyruvique, la molécule bifonctionnelle soit le glutaraldéhyde, le glyoxal, le succinaldéhyde, l'hexaméthylène-diisocyanate, le 2,4-diisocyanato-toluène ou un imido-ester bifonctionnel.

2. Composition suivant la revendication 1, caractérisée en ce que l'antigène polypeptidique ou glycoprotéinique est la gonadotrophine chorionique humaine.

3. Composition suivant l'une des revendications 1 et 2, caractérisée en ce que les érythrocytes sont stabilisés à l'aldéhyde pyruvique.

4. Composition suivant l'une quelconque des revendications 1 à 3, caractérisée en ce que la molécule bifonctionnelle est le glutaraldéhyde, l'hexaméthylène-diisocyanate ou le subérimidate de diméthyle.

5. Composition suivant l'une quelconque des revendications 1 à 4, caractérisée en ce qu'elle est lyophilisée.

6. Composition suivant l'une quelconque des revendications 1 à 5, caractérisée en ce que les érythrocytes sensibilisés à l'antigène ont été traites avec une solution physiologique salée tamponnée au phosphate contenant une protéine sérique dont le complément a été fixé au préalable et de laquelle les agglutinines non spécifiques ont été préalablement éliminées par adsorption en série avec des érythrocytes stabilisés à l'aldéhyde pyruvique.

7. Composition suivant l'une quelconque des revendications 1 à 5, caractérisée en ce que les érythrocytes stabilisés à l'antigène ont été traités avec une solution physiologique salée tamponnée au phosphate contenant une substance choisie entre la gélatine, des dextranes, la polyvinylpyrrolidone et des carboxyméthylcelluloses hydrosolubles qui a été préalablement traitée par adsorption avec des érythrocytes stabilisés à l'aldéhyde pyruvique.

8. Un procédé de préparation de compositions immunologiques d'érythrocytes d'animaux sensibilisés avec des antigènes utiles dans le test d'hémagglutination passive, qui consiste:

a) à recueillir le sang d'un animal directement dans un anticoagulant stérile isotonique de manière que le rapport final du sang à l'anticoagulant ait une valeur de 0,8:1 à 1,2:1 dans le mélange résultant, le volume cellulaire compact de concentration des érythrocytes dans le sang ayant une valeur de 15 à 25%;

b) à séparer les érythrocytes dudit mélange par centrifugation;

c) à laver les érythrocytes avec une solution physiologique salée normale, puis à les mettre en suspension dans la solution physiologique salée normale à une concentration de 10 à 30%;

d) à mélanger la suspension d'érythrocytes avec un mélange d'aldéhyde pyruvique, de solution tamponnée neutre et de solution physiologique salée normale pour obtenir un mélange à stabiliser;

e) à faire incuber le mélange à stabiliser de l'étape (d) à une température de 30 à 55°C pendant 1 à 5 heures et à recueillir les érythrocytes stabilisés par une centrifugation suivie d'un lavage à la solution physiologique salée normale;

f) à mettre en suspension les érythrocytes stabilisés dans une solution tamponnée neutre d'un antigène choisi entre la gonadotrophine chorionique, la gonadotrophine de sérum de jument gravide, l'antigène de carcinome embryonnaire, l'hormone lutéinisante, la folliculostimuline, la gonadotrophine humaine de la ménopause et l'hormone stimulant la thyroïde;

g) à former un mélange à sensibiliser par agitation correcte de la suspension d'érythrocytes stabilisés portant l'antigène avec une solution d'une molécule bifonctionnelle dans la solution physiologique salée, la molécule bifonctionnelle étant choisie entre le glutaraldéhyde, le glyoxal, le succinaldéhyde, hexaméthylène-diisocyanate, le 2,4-diisocyanato-toluène et un imido-ester bifonctionnel;

h) à recueillir par centrifugation les érythrocytes sensibilisés, puis à les laver avec une solution physiologique salée tamponnée neutre;

i) à mettre en suspension les érythrocytes sensibilisés dans une solution physiologique salée tamponnée neutre contenant du sérum normal d'un animal dont le complément a au préalable été fixé, ces érythrocytes étant des érythrocytes de mammifères, d'oiseaux ou de reptiles.

9. Un procédé suivant la revendication 8 pour la préparation d'une composition suivant la revendication 3, dans lequel l'antigène est la gonadotrophine chorionique humaine.

10. Un procédé suivant la revendication 8 ou 9 dans lequel la composition est lyophilisée.

11. Méthode de détection de la présence ou de l'absence de gonadotrophine chorionique humaine dans un échantillon d'urine humaine, caractérisée en ce que la composition suivant la revendication 3 dans laquelle l'antigène est la gonadotrophine chorionique humaine et les érythrocytes sont des érythrocytes de mammifère, d'oiseau ou de reptile, est mélangée avec un antisérum anti-hCG très purifié et avec de l'urine humaine concentrée par ultrafiltration, de sorte que s'il n'y a pas d'hCG, une agglutination des érythrocytes a lieu au repos, tandis que s'il y a de l'hCG, une telle agglutination n'apparaît pas.

12. Une composition d'antisérum anti-hCG destinée à être utilisée dans une méthode suivant la revendication 11, qui a été préparée

en utilisant comme antigène immunisant une hCG très purifiée, ladite composition ayant une réactivité croisée avec d'autres hormones glycoprotéiniques antigéniques de moins de 25%.

**Patentansprüche**

1. Immunologisches Mittel aus mit Pyruvaldehyd oder Dimethylsuberinsäureimidat stabilisierten Erythrocyten, die mit einem Polypeptid- oder Glocyproteinantigen, ausgewählt unter Gonadotrophinium chorionicum, Gonadotrophinium sericum trächtiger Stuten, Embroykarzinomantigen, luteinisierendem Hormon, follikelstimulierendem Hormon, menschlichem Menopausen-gonadotropin und schilddrüsenstimulierendem Hormon sensibilisiert sind, wobei die stabilisierten Erythrocyten an die genannten Antigene mit einem bifunktionellen Molekül, ausgewählt unter Glutaraldehyd, Glyoxal, Bernsteinsäurealdehyd, Hexamethylendiisocyanat, Toluol-2,4-diisocyanat, einem bifunktionellen Imidoester, Cyanurchlorid, tetrazotiertem o-Anisidin und einem wasserlöslichen Carbodiimid, gekuppelt sind, wobei jeweils Voraussetzung ist, daß wenn die Erythrocyten mit Dimethylsuberinsäureimidat stabilisiert sind, das bifunktionelle Molekül nicht Dimethylsuberinsäureimidat sein kann, und wenn die Erythrocyten mit Pyruvaldehyd stabilisert sind, das bifunktionelle Molekül Glutaraldehyd, Glyoxal, Bernsteinsäurealdehyd, Hexamethylen - diisocyanat, Toluol - 2,4 - diisocyanat oder ein bifunktioneller Imidoester ist.

2. Mittel nach Anspruch 1, worin das Polypeptid- oder Glycoprotein-Antigen menschliches Gonadotrophinium chorionicum ist.

3. Mittel nach Anspruche 1 oder 2, worin die Erythrocyten mit Pyruvaldehyd stabilisiert sind.

4. Mittel nach einem der Ansprüche 1 bis 3, worin das bifunktionelle Molekül Glutaraldehyd, Hexamethylendiisocyanat oder Dimethylsuberinsäure-imidat ist.

5. Mittel nach einem der Ansprüche 1 bis 4, wobei das Mittel lyophilisiert ist.

6. Mittel nach einem der Ansprüche 1 bis 5, worin die Antigen-sensibilisierten Erythrocyten mit phosphatgepufferter Kochsalzlösung, die ein Serumprotein enthält, in dem das Komplement zuvor fixiert wurde, und aus dem unspezifische Agglutinine vorher durch Reihenadsorption mit mit Pyruvaldehyd stabilisierten Erythrocyten entfernt wurden, behandelt sind.

7. Mittel nach einem der Ansprüche 1 bis 5, worin die Antigen-sensibilisierten Erythrocyten mit phosphatgepufferter Kochsalzlösung, enthaltend einen Bestandteil, ausgewählt unter Gelatine, Dextran, Polyvinylpyrrolidin und wasserlöslichen Carboxymethylcellulosen, die zuvor einer Adsorptionsbehandlung unter Verwendung von mit Pyruvaldehyd stabilisierten Erythrocyten unterworfen worden waren, behandelt sind.

8. Verfahren zur Herstellung immunologischer Mittel aus tierischen Erythrocyten, die mit Antigenen sensibilisiert sind und zum passiven Hämagglutinationstest brauchbar sind, wobei man:

a) das Tierblut direkt in einem isotonischen, sterilen Antikoagulans aufnimmt, so daß sich ein Endverhältnis von Blut zu Antikoagulans von 0,8:1 bis 1.2:1 in der erhaltenen Mischung ergibt, wobei die Erythrocytenkonzentration, bzw. das Volumen der gepackten Zellen des Blutes dieser Mischung 15 bis 25% beträgt;

b) die Erythrocyten durch Zentrifugieren aus dieser Mischung abtrennt;

c) die Erythrocyten mit normaler Kochsalzlösung wäscht und dann bei einer Konzentration von 10 bis 30% die Erythrocyten in normaler Kochsalzlösung suspendiert;

d) die Erythrocytensuspension mit einer Mischung von Pyruvaldehyd, neutraler gepufferter Lösung und normaler Kochsalzlösung mischt, um eine Stabilisierungsmischung zu erhalten;

e) Die Stabilisierungsmischung aus Stufe (d) bei 30 bis 55°C 1 bis 5 Stunden lang inkubiert und die stabilisierten Erythrocyten durch Zentrifugieren und anschließendes Washen mit normaler Kochsalzlösung sammelt;

f) die stabilisierten Erythrocyten in einer neutralen gepufferten Lösung eines Antigens, ausgewählt unter Gonadotrophinium chorionicum, Gonadotrophinium sericum trächtiger Stuten, Embryokarzinomantigen, luteinisierenden Hormonen, follikelstimulierenden Hormonen, menschlichem Menopausen-Gonadotrophinium und schilddrüsenstimulierenden Hormonen, suspendiert;

g) eine sensibilisierende Mischung herstellt, indem man die Suspension von stabilisiertem Erythrocyten-Antigen grundlich mit einer Lösung eines bifunktionellen Moleküls in Kochsalzlösung rührt, wobei das bifunktionelle Molekül ausgewäht ist unter Glutaraldehyd, Glyoxal, Bernsteinsäurealdehyd, Hexamethylen-diisocyanat, Toluol-2,4-diisocyanat und einem bifunktionellen Imidoester;

h) die sensibilisierten Erythrocyten durch Zentrifugieren sammelt und anschließend mit neutraler, gepufferter Kochsalzlösung wäscht;

i) die sensibilisierten Erythrocyten in einer neutralen gepufferten Kochsalzlösung suspendiert, welche normales Tierserum enthält worin das Komplement dieses Serums zuvor fixiert worden war, wobei die Erythrocyten von Sägetieren, Vögeln oder Reptilien stammen.

9. Verfahren nach Anspruch 8 zur Herstellung eines Mittels wie in Anspruch 3 beansprucht, wobei das Antigen menschliches Gonadotrophinium chorionicum ist.

10. Verfahren nach Anspruch 8 oder 9, wobei das Mittel lyophilisert ist.

11. Verfahren zur Bestimmung der An- oder Abwesenheit von menschlichem Gonadotrophinium chorionicum in einer Urinprobe von Menschen, dadurch gekennzeichnet, daß man das Mittel nach Anspruch 3, worin das Antigen menschliches Gonadotrophinium chorionicum ist und die Erythrocyten von Säugetieren,

Vögeln oder Reptilien stammen, mit einem hochreinen menschlichen Gonadotrophinium chorionicum Antiserum (hCG) und mit ultrafiltriertem konzentriertem menschlichen Urin mischt, wobei wenn kein menschliches Gonadotrophinium chorionicum anwesend ist, die Erythrocyten beim Stehen agglutinieren, während wenn menschliches Gonadotrophinium chorionicum anwesend ist, keine derartige Agglutination eintritt.

12. Antiserummittel für menschliches Gonadotrophinium chorionicum zur Verwendung in einem Verfahren nach Anspruch 11, welches hergestellt wurde unter Verwendung eines stark gereinigten menschlichen Gonadotrophinium chorionicum als immunisierendem Antigen, wobei die "cross-reactivity" dieses Mittels gegenüber anderen Glycoproteinhormon-Antigenen geringer als 25% ist.